# FASCICULE DE BREVET EUROPEEN

(11) **EP 2 465 425 B1**
(45) Date de publication et mention de la délivrance du brevet: **23.01.2013**
(21) Numéro de dépôt: 11182241.7
(22) Date de dépôt: 21.09.2011
(51) Int. Cl.: A61B 5/0215, A61B 5/042, A61N 1/05

(54) **Sonde pour dispositif médical implantable actif, comportant une puce électronique notamment pour le multiplexage**
Leitung für ein aktives implantierbares medizinisches Gerät, umfassend einen Chip, insbesondere zum Multiplexen
Lead for an active implantable medical device, comprising a microchip, in particular for multiplexing

(30) Priorité: 14.12.2010 FR 1060452
(43) Date de publication de la demande: 20.06.2012
(73) Titulaire: Sorin CRM SAS, 92140 Clamart Cedex (FR)
(72) Inventeur: Ollivier, Jean-François, 91190 Villiers le Bacle (FR)
(74) Mandataire: Dupuis-Latour, Dominique

(56) Documents cités:
- EP-A1- 1 036 572
- WO-A2-2006/069322
- WO-A2-2010/091435
- US-A1- 2002 161 422

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les pompes de diffusion de substances médicales, les implants cochléaires, les capteurs biologiques implantés, ainsi que les dispositifs de mesure de pH ou encore d'impédance intracorporelle (telle que mesure d'impédance transpulmonaire ou d'impédance intracardiaque).

Pour le recueil des signaux et la délivrance d'impulsions de stimulation, ces dispositifs médicaux implantables actifs font appel à des électrodes intégrées à une sonde, elle-même reliée au générateur du dispositif.

Ces électrodes sont destinées à venir en contact avec les tissus à stimuler ou ceux sur lesquels on souhaite recueillir un signal électrique : myocarde, nerf, muscle. Dans le cas d'un dispositif de diagnostic et de thérapie cardiaque, ces électrodes peuvent être des électrodes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

Un premier aspect du développement de ces appareils est la multiplication du nombre d'électrodes, notamment pour les appareils dits "multisite" qui permettent de choisir les sites de stimulation/détection optimisant le fonctionnement de l'appareil.

La multiplication des électrodes peut également résulter de la présence à un même niveau de la sonde de plusieurs électrodes sectorielles (électrodes dirigées spécifiquement dans une direction radiale par rapport à la sonde, au site de stimulation), avec possibilité de sélectionner l'une ou l'autre de ces électrodes sectorielles afin d'optimiser la délivrance des impulsions au site choisi. Il en est ainsi notamment des sondes implantées dans le réseau coronaire veineux, destinées à la stimulation indirecte d'une cavité gauche : avec plusieurs électrodes sectorielles, il est aisé de choisir celle qui est tournée vers la paroi de l'épicarde, face à la cavité, et en contact avec cette paroi et d'éviter ainsi la stimulation du nerf phrénique.

Un autre aspect du développement des appareils implantables est l'intégration à la sonde de capteurs divers, tout particulièrement de capteurs de pression sanguine ou d'accélération, notamment d'accélération endocardiaque (EA). Les signaux recueillis par ces capteurs donnent une information représentative de l'état hémodynamique instantané du patient, permettant un pilotage très efficace des diverses fonctions du dispositif.

Ces capteurs de sonde requièrent également une liaison spécifique pour la transmission des signaux depuis le capteur, situé à l'extrémité distale de la sonde, jusqu'au générateur connecté à l'extrémité opposée, proximale. Cette liaison propre au capteur vient se surajouter aux liaisons spécifiques déjà existantes entre le générateur et les diverses électrodes situées en la région distale de la sonde.

Dans tous les cas, compte tenu du diamètre très restreint du corps de sonde, il n'est pas envisageable d'y loger autant de conducteurs que d'électrodes, ce qui conduirait aussi bien à des dimensions inacceptables qu'à des difficultés de réalisation, notamment au niveau de la connectique de liaison de la sonde au générateur implanté auquel elle est couplée. Ces développements imposent donc la mise en place de circuits électroniques de multiplexage permettant de gérer l'échange des signaux multiples entre la sonde (électrodes et/ou capteurs) et le générateur, et inversement. Le multiplexage est assuré *in situ,* à proximité des électrodes, par un circuit électronique (ci-après simplement appelé "puce") incorporé à la sonde dans la partie distale de celle-ci, à son extrémité ou dans une région voisine (là où par exemple les impulsions doivent être délivrées). Les EP 1 938 861 A1 et le EP 2 082 684 A1 (tous les deux au nom de ELA Medical) décrivent une telle sonde munie d'une pluralité d'électrodes au voisinage de son extrémité distale, par exemple dix électrodes, associées à une puce, encapsulée de manière hermétique au voisinage des électrodes dans un anneau rigide, assurant le multiplexage/démultiplexage des électrodes avec un bus commun formé par deux conducteurs isolés s'étendant sur toute la longueur de la sonde jusqu'au connecteur proximal permettant le couplage avec le générateur, ce dernier étant équipé d'un circuit homologue de démultiplexage/multiplexage.

Les WO 2010/091435 A2 et WO 2006/069322 A2 (Proteus Biomedical, Inc.) décrivent un système dans lequel un bus bifilaire commun véhicule des signaux en provenance/à destination d'électrodes sectorielles adressables formées sur des "satellites" portés par la sonde à intervalles réguliers. La construction décrite ne laisse cependant subsister pour la sonde qu'une lumière interne de diamètre très inférieur au diamètre extérieur, en raison de l'encombrement de la puce et des liaisons aux fils. Cette contrainte limite l'application d'une telle technique à des électrodes situées en bout de sonde, car sinon le faible diamètre de la lumière ne permettrait pas l'emploi de techniques de pose classiques, à moins d'augmenter fortement le diamètre extérieur de la sonde à l'endroit des électrodes, ce qui en limiterait les applications. En outre, cette technologie de réalisation est extrêmement délicate à mettre en oeuvre et sa fiabilité dans le temps n'a pas été prouvée.

L'un des buts de l'invention est de proposer une sonde qui remédie aux inconvénients précités, avec :
- une lumière interne de diamètre comparable à celui des sondes existantes actuelles (typiquement un diamètre interne de 0,55 mm, soit 1,65 French), présentant en outre un isolement électrique garanti par rapport aux conducteurs et à la puce ;
- un diamètre extérieur lui aussi comparable à ce qui existe aujourd'hui (typiquement un diamètre extérieur de 1,6 mm, soit 4,8 French, voire même 4 French), avec un diamètre constant sur toute la longueur de la sonde, c'est-à-dire une configuration de sonde dite "monodiamètre" ;
- une puce encapsulée de façon native grâce à la géométrie et la configuration des éléments de la sonde, avec un excellent isolement électrique et une protection mécanique à l'encontre des contraintes aussi bien en torsion qu'en flexion ;
- une sonde résultante présentant pour le praticien une configuration apparente conventionnelle, autorisant une mise en place sans changement des techniques de pose habituelles ;
- enfin, du point de vue de la technologie, une sonde qui puisse être fabriquée par mise en oeuvre de processus standard dans le domaine de la fabrication des sondes, donc de processus éprouvés susceptibles d'être mis en oeuvre à moindre coût.

L'invention propose à cet effet une sonde du type connu divulgué notamment par le WO 2010/091435 A2 précité, comportant : une gaine flexible allongée avec une lumière centrale ; au moins une liaison conductrice logée dans la lumière de la gaine ; un tube support cylindrique rigide en matériau isolant, avec un alésage central traversant, la gaine étant interrompue par le tube support qui est intercalé sur la gaine de manière que son alésage soit coaxial avec la lumière de la gaine, le tube support comportant une cavité formant réceptacle de puce, débouchant dans une région centrale du tube support en surface de celui-ci ; au moins une électrode de détection/stimulation portée par le tube support au niveau de la surface de la gaine ; et au moins un circuit électronique, comportant sur un substrat une puce et au moins deux plages conductrices de contact reliées respectivement à la liaison conductrice et à l'électrode, la puce étant disposée avec une conformation courbée dans le réceptacle du tube support, la puce portant sur une face du substrat une plage conductrice extérieure et sur la face opposée une plage conductrice intérieure, l'électrode de détection/stimulation étant électriquement reliée à la plage conductrice extérieure de la puce ; et un conducteur de traversée relié dans une région centrale à la plage conductrice intérieure de la puce.

De façon caractéristique de l'invention, le substrat de la puce est un substrat flexible, la liaison conductrice logée par la gaine comporte une discontinuité à l'endroit du tube support, et le conducteur de traversée est une bande conductrice formée en surface du tube support, elle s'étend en direction axiale le long de celui-ci, et elle est reliée à chacune de ses extrémités à la liaison conductrice en vis-à-vis logée dans la gaine.

Par ailleurs, la puce est une puce sur substrat flexible, portant sur une face du substrat une plage conductrice extérieure et sur la face opposée une plage conductrice intérieure, et cette puce est disposée avec une conformation courbée dans le réceptacle du tube support. L'électrode de détection/stimulation est portée par le tube support et elle est électriquement reliée à la plage conductrice extérieure de la puce. Quant à la bande conductrice, elle est reliée (i) à chacune de ses extrémités, à la liaison conductrice en vis-à-vis logée dans la gaine, et (ii) dans une région centrale, à la plage conductrice intérieure de la puce.

Selon diverses caractéristiques subsidiaires avantageuses:
- le matériau isolant du tube support est une céramique ;
- la sonde comporte une unique électrode annulaire en forme de manchon conducteur s'étendant en direction circonférentielle sur tout le pourtour du tube support et enfilé sur la région centrale de celui-ci ;
- la sonde comporte une pluralité d'électrodes sectorielles isolées les unes des autres, réparties en direction circonférentielle sur le pourtour du tube support et reliées chacune à une pluralité correspondante de plages conductrices extérieures de la puce ;
- le diamètre hors-tout de la sonde est constant dans la région du tube support et de l'électrode, et égal au diamètre de la gaine flexible ;
- la sonde comporte deux liaisons conductrices distinctes logées dans la lumière de la gaine, le tube support comportant deux bandes conductrices de traversée distinctes correspondantes, isolées entre elles et formées dans des régions diamétralement opposées du tube support.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
Les Figures 1 à 8 illustrent les étapes successives de fabrication d'une sonde selon l'invention dans sa partie incluant la puce et l'électrode, avec ses différents éléments constitutifs, la Figure 8 présentant la partie en question de la sonde, dans son état achevé.
Les Figures 9 à 11 sont homologues des figures précédentes, pour une variante de réalisation incluant plusieurs électrodes sectorielles.

On va maintenant décrire un exemple de réalisation du dispositif de l'invention.

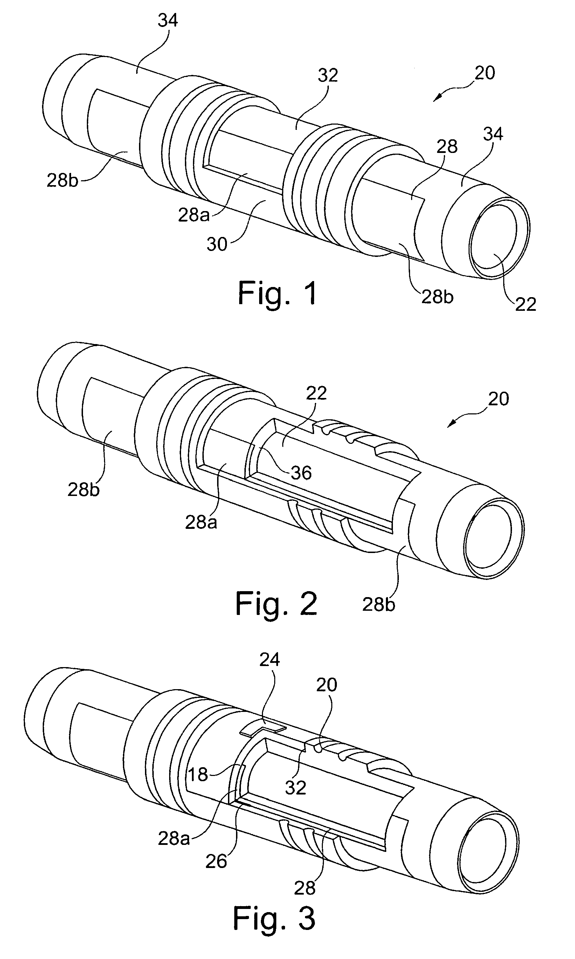

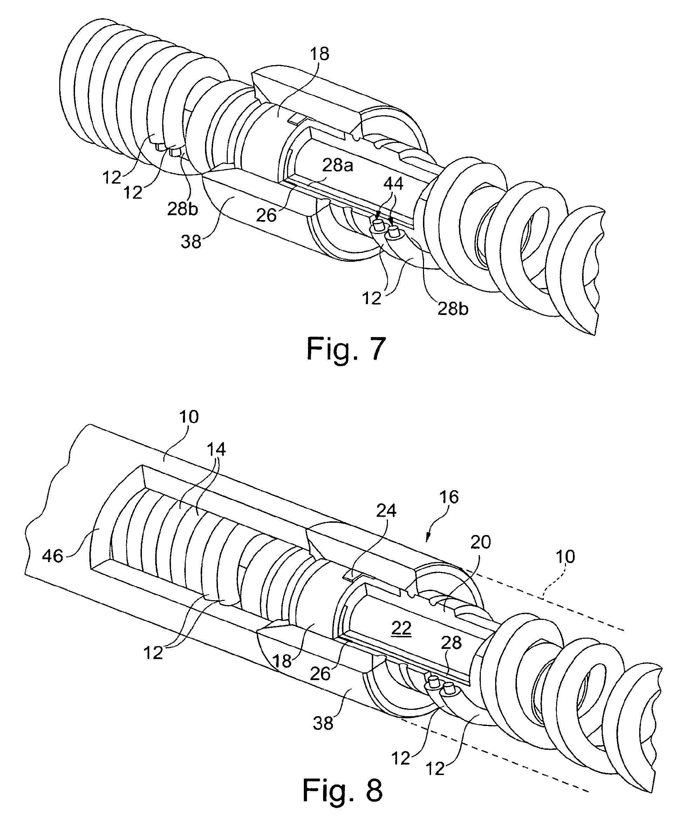

La Figure 8 représente la partie de la sonde caractéristique de l'invention avec ses différents éléments constitutifs, telle qu'obtenue après exécution des différentes étapes de fabrication illustrées Figures 1 à 7.

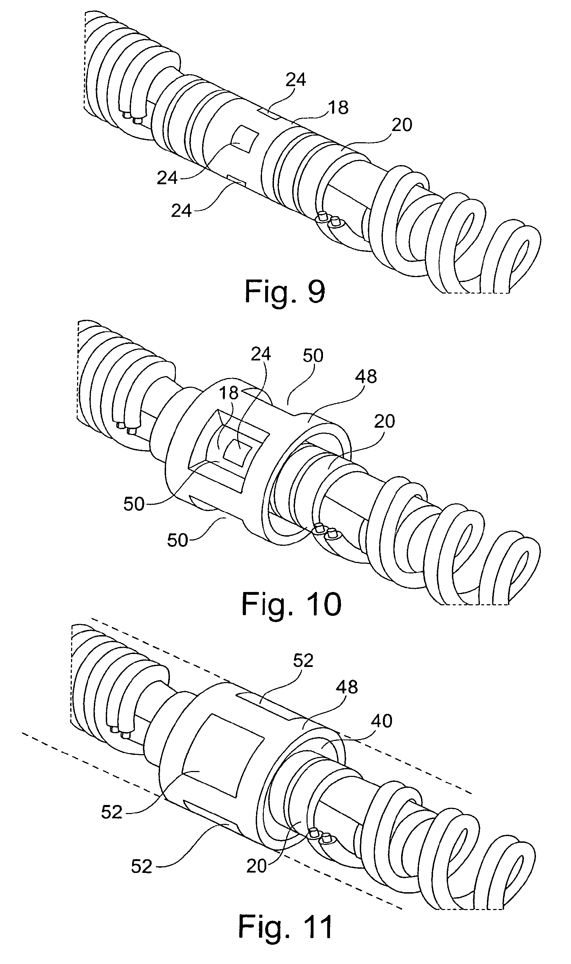

La référence 10 désigne de façon générale le corps d'une sonde de type général connu, dont on n'a représenté sur les figures qu'une partie, généralement située au voisinage des extrémités distales, portant une électrode de recueil et/ou de stimulation, cette électrode étant une électrode annulaire dans l'exemple illustré Figures 1 à 8 (et une pluralité d'électrodes sectorielles dans l'exemple illustré Figures 9 à 11, décrit plus bas). La sonde inclut deux liaisons conductrices en forme de fil spiralé individuellement isolé, chacune des deux liaisons étant par exemple constituée d'une paire respective de conducteurs 12,12 et 14,14. Ces liaisons conductrices courent sur toute la longueur de la sonde et sont reliées, côté distal, à un connecteur de couplage à un générateur implant (non représenté). La sonde porte également une électrode annulaire 16 reliée à une puce 18 assurant le multiplexage/démultiplexage de cette électrode (de même que d'autres électrodes situées à d'autres endroits de la sonde) avec les liaisons conductrices 12, 14 jouant le rôle de bus de liaison entre les diverses électrodes de la sonde et le générateur distant.

La puce 18 est donc reliée électriquement, d'une part, à l'électrode 16 et, d'autre part, à chacune des deux liaisons conductrices 12 et 14.

Par commodité, et pour la simplicité de l'exemple, la puce 18 est ici décrite sous forme d'un multiplexeur/démultiplexeur d'électrode(s), mais l'invention n'est pas limitée à ce type de circuit : la puce 18 peut aussi bien être couplée à un capteur (c'est-à-dire un transducteur de signal permettant de produire un signal électrique fonction des variations d'un paramètre physique), ou même incorporer un tel capteur, ou encore comprendre un circuit électronique actif tel qu'amplificateur, filtre associé ou non à un capteur disposé à proximité, ou encore un microsystème électromécanique (MEMS), ou encore de façon générale tout élément actif technologiquement intégrable dans un corps de sonde.

La puce comprend deux sorties correspondant aux deux liaisons conductrices 12 et 14 du bus bifilaire, ainsi qu'une troisième sortie correspondant à la liaison à l'électrode 16, et éventuellement des sorties supplémentaires dans le cas par exemple d'une configuration multi-électrodes (comme dans le cas des Figures 9 à 11 que l'on décrira plus bas, comprenant une pluralité d'électrodes sectorielles multiplexées par une même puce).

De façon caractéristique de l'invention, la puce est montée sur un tube support cylindrique rigide 20 en matériau isolant, comportant un alésage central traversant 22 permettant d'assurer, sans réduction de diamètre, la continuité de la lumière interne de la sonde, nécessaire par exemple au passage d'un mandrin ou d'un passe-fil lors de la pose.

Pour permettre le montage sur ce tube support cylindrique, la puce est une puce de type à substrat flexible, c'est-à-dire dont le substrat est suffisamment aminci pour pouvoir être courbé et épouser la surface cylindrique du support. Cette technique est par exemple décrite par Zimmermann et al., A Seamless Ultra-Thin Chip Fabrication and Assembly Process, Electron Devices Meeting IEDM '06, 11-13 Déc. 2006, pp. 1-3. Pour rendre la puce flexible, il est nécessaire d'amincir son substrat (généralement un substrat en silicium) à une épaisseur inférieure à 0,1 mm pour qu'elle puisse épouser la forme du tube, dont le diamètre extérieur est, dans l'exemple illustré, de 0,85 mm à l'endroit où est située la puce.

La puce porte sur sa surface externe (convexe) une plage conductrice externe 24 destinée à venir en contact avec l'électrode 16, et elle porte à sa surface opposée (concave) deux plages conductrices internes 26 diamétralement opposées (une seule de ces plages 26 étant visible sur les figures), destinées à être reliées, de la manière que l'on décrira plus bas, aux deux liaisons conductrices respectives 12 et 14. Ces diverses plages conductrices comportent par exemple un alliage d'or pouvant être brasé par refusion à 450°C pour établir les liaisons électriques requises avec les éléments que l'on vient d'indiquer. Les Figures 1 et 2 montrent, isolément, le tube support 20 avant que celui-ci ne reçoive la puce flexible (la Figure 2 est une vue écorchée de la Figure 1, montrant en section la structure interne du tube).

Ce tube est un tube en matériau électriquement isolant, réalisé par surmoulage de matière plastique ou par assemblage de composants en céramique solidarisés par refusion à haute température ou par collage. Il porte deux bandes conductrices allongées 28, diamétralement opposées (une seule de ces bandes étant visible sur les figures) formées en surface et s'étendant en direction axiale sur la majeure partie de la longueur du tube de manière à former une traversée, avec une région centrale 28a apparente et deux régions d'extrémité 28b.

Le tube support comporte une région centrale 30 de diamètre supérieur, par exemple 1 mm, pourvue d'une cavité 32 formant réceptacle pour la puce flexible. La profondeur de cette cavité correspond sensiblement à l'épaisseur de la puce de sorte que celle-ci, lorsqu'elle sera mise en place, viendra affleurer le contour de la cavité (comme illustré Figure 3). La configuration du tube est telle que la partie centrale 28a de la bande de traversée 28 est apparente au fond de la cavité 32.

Le tube support comporte également deux régions d'extrémité 34 de moindre diamètre, par exemple 0,85 mm dans l'exemple illustré, portant les deux extrémités respectives 28b de la bande conductrice de traversée 28. La longueur du tube est par exemple de l'ordre de 5 mm et la surface du réceptacle de l'ordre de 1 à 2 mm².

On notera que l'alésage central 22 est sur le plan électrique totalement isolé des bandes de traversée 28 et de la cavité 32, grâce à une épaisseur de paroi 36 (Figure 2) du tube support, qui permet de préserver l'isolement électrique tout en conservant un diamètre d'alésage relativement important, de l'ordre de 0,55 mm.

La Figure 3 illustre le tube support après report de la puce 18 dans la cavité 32.

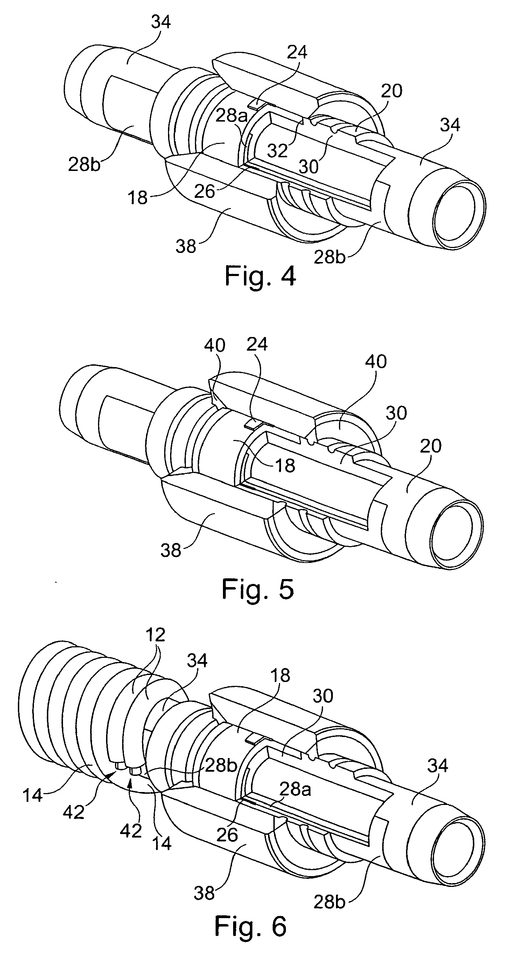

L'étape suivante, illustrée Figure 4, consiste à enfiler sur l'ensemble précédemment obtenu un manchon 38 en matériau conducteur, ajusté sur la partie centrale du tube support 20 et reposant sur toute l'étendue, en direction axiale, de la cavité 32, de sorte que ce manchon 38 couvre la totalité de la surface de la puce 18.

Une étape de refusion thermique permet d'établir la liaison électrique entre chacune des plages conductrices intérieures 26 de la puce et la partie centrale 28a de la bande conductrice de traversée 28, permettant ainsi d'assurer une continuité électrique entre chacune de ces plages et les deux extrémités 28b parentes de 28 aux extrémités 34 du tube support 20. Ce chauffage permet également d'établir le contact électrique entre la plage externe 24 de la puce 18 et le matériau conducteur du manchon 38, destiné à constituer l'électrode annulaire 16.

L'étape suivante, illustrée Figure 5, consiste à parfaire l'étanchéité de l'ensemble à l'égard de l'environnement extérieur, en injectant une masse 40 de matériau, par exemple de colle polyuréthanne, dans l'intervalle entre le manchon 38 et la région centrale 30 du tube support 20. Outre l'étanchéité, cette masse de colle permet de protéger l'ensemble, et notamment la puce 18, à l'égard de toutes les contraintes et sollicitations mécaniques externes, assurant ainsi une encapsulation protectrice et durable de l'ensemble des éléments électriques et électroniques, y compris pendant l'assemblage de la sonde en usine.

A ce stade, l'ensemble obtenu est prêt pour une inspection visuelle, un test des fonctionnalités électriques avant l'assemblage sur le corps de sonde que l'on va maintenant décrire.

L'étape suivante, illustrée Figure 6, consiste à mettre en place des liaisons électriques formées par les conducteurs spiralés 12,12 et 14,14 et à souder les conducteurs de chacune de ces liaisons à la plage 28b correspondante, par exemple au moyen d'une soudure laser 42,42 entre l'extrémité dénudée des conducteurs 12,12 et la plage d'extrémité 28b côté proximal de la bande conductrice de traversée située à cet endroit. La même soudure est effectuée dans la région diamétralement opposée (non visible sur la figure) entre les conducteurs 14,14 de l'autre liaison électrique et l'autre bande conductrice de traversée diamétralement opposée à celle visible sur la Figure 6.

L'étape suivante, illustrée Figure 7, consiste à effectuer la même opération du côté opposé du manchon annulaire 38, c'est-à-dire côté distal, par exemple par soudure laser 44,44 des conducteurs 12,12 sur l'extrémité 28b côté distal de la bande conductrice de traversée 28 (et de la même façon pour les conducteurs 14,14 dans la région diamétralement opposée).

Une fois ces soudures réalisées, la continuité électrique des liaisons conductrices 12,12 et 14,14 est assurée de part et d'autre du manchon 38. D'autre part, une connexion a été établie, comme on l'a expliqué en référence à la Figure 4, avec chacune de ces liaisons et la plage intérieure correspondante de la puce 18, par exemple, comme illustré, entre la liaison 12,12 et la plage intérieure 26.

L'étape finale, illustrée Figure 8, consiste à enrober les conducteurs spiralés 12,12 et 14,14 de part et d'autre du manchon 38, par une gaine isolée cylindrique, par exemple en polyuréthanne ou en silicone, sur un diamètre constant, identique à celui du manchon 38.

On dispose ainsi d'une sonde de type monodiamètre, d'un diamètre extérieur typique de 1,6 mm (4,8 French) identique à celui des sondes conventionnelles, par exemple une sonde de type *Xfine TX26D* fabriquée par Sorin CRM, Clamart, France, et pourvue d'une lumière interne 22 de 0,55 mm au moins (diamètre de l'alésage central du tube support 20).

En disposant autant de tubes supports 20 et de manchons conducteurs 38 qu'on souhaite d'électrodes annulaires successives, il est ainsi possible de réaliser une sonde comportant en différents endroits sur sa longueur une pluralité d'électrodes, toutes multiplexées au moyen d'une puce respective correspondante située sous l'électrode.

Les Figures 9 à 11 illustrent une variante dans laquelle le manchon conducteur 38 qui formait l'électrode annulaire 16 du mode de réalisation précédent est remplacé par un manchon isolant 48 de même diamètre, mais pourvu d'une pluralité de cavités 50, par exemple quatre cavités 50 axialement orientées, laissant apparaître chacune une plage conductrice extérieure 24 de la puce 18. Il s'agit de réaliser, au lieu d'une électrode annulaire unique, une pluralité d'électrodes sectorielles, par exemple orientées selon quatre quadrants, ces électrodes étant sélectionnables à volonté au moyen d'un système de multiplexage intégré à la puce 18. Cette dernière est donc pourvue d'une pluralité de plages conductrices externes 24, en nombre égal à celui des électrodes sectorielles, ces plages restant apparentes au fond des cavités 50 du manchon 48 après mise en place de celui-ci sur le tube support 20 (Figure 10).

Les électrodes sectorielles 52 sont formées par exemple en clipsant un composant micromécanique conducteur venant en contact avec la plage conductrice extérieure 24 de la puce 18, et dont la surface extérieure (qui affleure le manchon cylindrique 48) constitue l'électrode sectorielle proprement dite.

On notera qu'il est aisé d'ajuster la surface et la forme de chacune des électrodes sectorielles, simplement en définissant de la manière souhaitée les dimensions et la forme des cavités 50 du manchon isolant 48.

## Revendications

1. Une sonde pour un dispositif médical implantable actif, comportant :
- une gaine flexible allongée (10) avec une lumière centrale ;
- au moins une liaison conductrice (12, 14) logée dans la lumière de la gaine ;
- un tube support cylindrique rigide (20) en matériau isolant, avec un alésage central traversant (22), la gaine étant interrompue par le tube support qui est intercalé sur la gaine de manière que son alésage soit coaxial avec la lumière de la gaine,
- le tube support comportant une cavité formant réceptacle (32) de puce, débouchant dans une région centrale (30) du tube support en surface de celui-ci ;
- au moins une électrode de détection/stimulation (16 ; 52) portée par le tube support (20) au niveau de la surface de la gaine ; et
- au moins un circuit électronique, comportant sur un substrat une puce (18) et au moins deux plages conductrices de contact (26, 24) reliées respectivement à la liaison conductrice et à l'électrode, la puce étant disposée avec une conformation courbée dans le réceptacle (32) du tube support,
la puce (18) portant sur une face du substrat une plage conductrice extérieure (24) et sur la face opposée une plage conductrice intérieure (26), l'électrode de détection/stimulation (16 ; 52) étant électriquement reliée à la plage conductrice extérieure (24) de la puce ; et
- un conducteur de traversée (28) relié dans une région centrale (28a) à la plage conductrice intérieure (26) de la puce,
sonde **caractérisée en ce que** :
- le substrat de la puce (18) est un substrat flexible ;
- la liaison conductrice logée par la gaine comporte une discontinuité à l'endroit du tube support ; et
- le conducteur de traversée est une bande conductrice (28) formée en surface du tube support, elle s'étend en direction axiale le long de celui-ci, et elle est reliée à chacune de ses extrémités (28b) à la liaison conductrice en vis-à-vis (12) logée dans la gaine.

2. La sonde de la revendication 1, dans laquelle le matériau isolant du tube support est une céramique.

3. La sonde de la revendication 1, comportant une unique électrode annulaire en forme de manchon conducteur (16) s'étendant en direction circonférentielle sur tout le pourtour du tube support et enfilé sur la région centrale (30) de celui-ci.

4. La sonde de la revendication 1, comportant une pluralité d'électrodes sectorielles (52) isolées les unes des autres, réparties en direction circonférentielle sur le pourtour du tube support et reliées chacune à une pluralité correspondante de plages conductrices extérieures (24) de la puce.

5. La sonde de la revendication 1, dans laquelle le diamètre hors-tout de la sonde est constant dans la région du tube support et de l'électrode, et égal au diamètre de la gaine flexible.

6. La sonde de la revendication 1, comportant deux liaisons conductrices distinctes (12, 14) logées dans la lumière de la gaine, le tube support comportant deux bandes conductrices de traversée (28) distinctes correspondantes, isolées entre elles et formées dans des régions diamétralement opposées du tube support.

## Claims

1. Probe for an active implantable medical device, comprising:
- an elongate flexible sheath (10) with a central opening;
- at least one conductive link (12, 14) housed in the opening of the sheath;
- a rigid cylindrical support tube (20) made of insulating material, with a through central bore (22), the sheath being interrupted by the support tube which is inserted on the sheath so that its bore is coaxial with the opening of the sheath,
- the support tube comprising a cavity forming a chip receptacle (32), opening into a central region (30) of the support tube on the surface thereof;
- at least one detection/stimulation electrode (16; 52) borne by the support tube (20) on the surface of the sheath; and
- at least one electronic circuit, comprising, on a substrate, a chip (18) and at least two conductive contact lands (26, 24) linked respectively to the conductive link and to the electrode, the chip being arranged with a curved conformation in the receptacle (32) of the support tube,
the chip (18) bearing, on one face of the substrate, an external conductive land (24) and, on the opposite face, an internal conductive land (26), the detection/stimulation electrode (16; 52) being electrically linked to the external conductive land (24) of the chip; and
- a through conductor (28) linked in a central region (28a) to the internal conductive land (26) of the chip,
the probe being **characterized in that**:
- the substrate of the chip (18) is a flexible substrate;
- the conductive link housed by the sheath comprises a discontinuity at the position of the support tube; and
- the through conductor is a conductive strip (28) formed on the surface of the support tube, it extends in an axial direction along the latter, and it is linked at each of its ends (28b) to the facing conductive link (12) housed in the sheath.

2. Probe of Claim 1, in which the insulating material of the support tube is a ceramic.

3. Probe of Claim 1, comprising a single annular electrode in the form of a conductive sleeve (16) extending circumferentially all around the support tube and threaded over the central region (30) thereof.

4. Probe of Claim 1, comprising a plurality of sector-based electrodes (52) insulated from one another, distributed circumferentially all around the support tube and each linked to a corresponding plurality of external conductive lands (24) of the chip.

5. Probe of Claim 1, in which the overall diameter of the probe is constant in the region of the support tube and of the electrode, and equal to the diameter of the flexible sheath.

6. Probe of Claim 1, comprising two distinct conductive links (12, 14) housed in the opening of the sheath, the support tube comprising two corresponding distinct through conductive strips (28), insulated from one another and formed in diametrically opposite regions of the support tube.

## Patentansprüche

1. Sonde für eine aktive implantierbare medizinische Vorrichtung, die Folgendes aufweist:
- eine längliche flexible Hülle (10) mit einem zentralen Langloch;
- mindestens eine leitende Verbindung (12, 14), die im Langloch der Hülle untergebracht ist;
- ein steifes zylindrisches Tragrohr (20) aus Isoliermaterial mit einer zentralen Durchgangsbohrung (22), wobei die Hülle durch das Tragrohr unterbrochen wird, das so auf die Hülle zwischengeschoben wird, dass seine Bohrung mit dem Langloch der Hülle koaxial ist,
- wobei das Tragrohr einen Hohlraum aufweist, der einen Aufnahmebehälter (32) für einen Chip formt, der in einer zentralen Zone (30) des Tragrohrs an dessen Oberfläche mündet;
- mindestens eine Erfassungs-/Stimulationselektrode (16; 52), die vom Tragrohr (20) im Bereich der Oberfläche der Hülle getragen wird; und
- mindestens eine elektronische Schaltung, die auf einem Substrat einen Chip (18) und mindestens zwei leitende Kontaktbereiche (26, 24) aufweist, die mit der leitenden Verbindung bzw. mit der Elektrode verbunden sind, wobei der Chip mit einer gekrümmten Gestaltung im Aufnahmebehälter (32) des Tragrohrs angeordnet ist,
wobei der Chip (18) auf einer Seite des Substrats einen äußeren leitenden Bereich (24) und auf der gegenüberliegenden Seite einen inneren leitenden Bereich (26) trägt, wobei die Erfassungs-/Stimulationselektrode (16; 52) elektrisch mit dem äußeren leitenden Bereich (24) des Chips verbunden ist; und
- einen Durchgangsleiter (28), der in einer zentralen Zone (28a) mit dem inneren leitenden Bereich (26) des Chips verbunden ist,
wobei die Sonde **dadurch gekennzeichnet ist, dass**:
- das Substrat des Chips (18) ein flexibles Substrat ist,
- die in der Hülle untergebrachte leitende Verbindung eine Unterbrechung an der Stelle des Tragrohrs aufweist; und
- der Durchgangsleiter ein leitendes Band (28) ist, das an der Oberfläche des Tragrohrs geformt ist, es erstreckt sich in axialer Richtung entlang von diesem, und es ist an jedem seiner Enden (28b) mit der gegenüberliegenden leitenden Verbindung (12) verbunden, die in der Hülle untergebracht ist.

2. Sonde nach Anspruch 1, bei der das Isoliermaterial des Tragrohrs ein Keramikmaterial ist.

3. Sonde nach Anspruch 1, die eine einzige Ringelektrode in Form einer leitenden Muffe (16) aufweist, die sich in Umfangsrichtung auf dem ganzen Umfang des Tragrohrs erstreckt und auf dessen zentrale Zone (30) aufgeschoben ist.

4. Sonde nach Anspruch 1, die eine Vielzahl von Sektorelektroden (52) aufweist, die voneinander isoliert, in Umfangsrichtung auf dem Umfang des Tragrohrs verteilt und je mit einer entsprechenden Vielzahl von äußeren leitenden Bereichen (24) des Chips verbunden sind.

5. Sonde nach Anspruch 1, bei der der Gesamtdurchmesser der Sonde in der Zone des Tragrohrs und der Elektrode konstant und gleich dem Durchmesser der flexiblen Hülle ist.

6. Sonde nach Anspruch 1, die zwei unterschiedliche leitende Verbindungen (12, 14) aufweist, die im Langloch der Hülle untergebracht sind, wobei das Tragrohr zwei entsprechende unterschiedliche leitende Durchgangsbänder (28) aufweist, die zueinander isoliert und in diametral entgegengesetzten Zonen des Tragrohrs geformt sind.
